# EUROPEAN PATENT APPLICATION

(11) **EP 4 269 585 A1**
(43) Date of publication of application: **01.11.2023**
(21) Application number: 21911066.5
(22) Date of filing: 24.12.2021
(51) Int. Cl.: C12N 15/113, A61K 35/76, A61K 48/00, C12N 1/15, C12N 1/19, C12N 1/21, C12N 5/10, C12N 15/09, C12N 15/63, C12P 19/34

(54) **GUIDE RNA FOR EDITING POLYADENYLATION SIGNAL SEQUENCE OF TARGET RNA**

(30) Priority: 25.12.2020 JP 2020216787
(71) Applicant: Astellas Pharma Inc., Chuo-ku Tokyo 103-8411 (JP)
(72) Inventor: ARAI, Takatomo, Tokyo 103-8411 (JP); MANDA, Mariko, Tokyo 103-8411 (JP)
(74) Representative: Hoffmann Eitle
(86) International application number: PCT/JP2021/048231
(87) International publication number: WO 2022/138929

(57) **Abstract**

An object of the present invention is to provide an antisense guide RNA for editing a polyadenylation signal sequence of a target RNA and a nucleic acid that encodes the guide RNA, which are expected to control expression of the target RNA with high efficiency. The present inventors have intensively examined technologies for controlling expression of a target RNA with high efficiency, thereby completing the present invention by discovering a guide RNA for ADAR-dependent editing of the target RNA, including an antisense region complementary to a portion of the target RNA including a polyadenylation signal sequence.

## Description

### TECHNICAL FIELD

The present invention relates to a guide RNA for recruiting an ADAR and editing a polyadenylation signal sequence.

### BACKGROUND ART

Gene expression control is an important function for maintaining cell homeostasis and adapting to changes in intracellular and extracellular environments. As shown in basic principle of a central dogma, genetic information is basically transmitted in order of DNA, mRNA, and protein. Expression/translation control at each stage is important to determine a final protein amount.

In eukaryotes, a precursor mRNA (pre-mRNA) of a messenger RNA (mRNA) transcribed and synthesized in a nucleus undergoes processing referred to as addition of a 5' cap structure, splicing, and addition of a poly-A strand (polyadenylation) to a 3' terminal, and a mature mRNA is synthesized and exported from the nucleus to a cytoplasm.

Polyadenylation of the 3' terminal of the mRNA requires a polyadenylation signal (PAS) present upstream 10 to 30 bases of a site in which a polyadenylic acid (poly (A)) is added, and a region having a high content of GU sequences present downstream 20 to 40 bases of PAS. PAS is a conserved motif of 6 bases, and as representative examples, AAUAAA and a single base substituent thereof, AUUAAA are known. This process is known as a reaction in which at least 20 or more factors are related, in addition to a cleavage-polyadenylation specificity factor including endonuclease (CPSF) or polyadenylate polymerase (PAP) which is a poly-A addition enzyme (Mol. Cell, 2009, Vol. 33, p. 365-376).

Regarding relevance to human diseases, in β-thalassemia which causes β-globin production disorders due to β-globin gene mutations or deletions, two mutations of β-globin PAS point mutations (AATAAA→AATAAG) and 5 base pair deletions (AATAAA→A-----) have been identified, these are not normally polyadenylated, and thus an mRNA amount is decreased (Proc. Natl. Acad. Sci. USA, 1992, Vol. 89, p. 4324-4328). Thus, PAS sequences also play an important role in control of polyadenylation in human diseases.

Control of translation efficiency is generally known as a role of a poly-A strand of mRNA. mRNA forms a cyclic structure by interaction between eukaryotic initiation factor 4E (eIF4E) binding to a 5' terminal cap structure and a poly (A) binding protein (Pablp) binding to a poly-A strand (EMBO J., 1996, Vol. 15, p.7168-7177). It is believed that this cyclic structure makes it easier for ribosomes dissociated from mRNA to be recruited again to the 5' terminal of mRNA after translation termination, thereby improving translation efficiency (eLife, 2017, Vol. 6, p. e25237). In addition, since a factor having a long poly-A strand of the same mRNA shows high translation efficiency, the importance of the poly-A strand length is shown in control of translation efficiency (RNA, 1998, Vol. 4, p. 1321-1331).

As another role of the poly-A strand of mRNA, involvement in mRNA stability is known. mRNA degradation pathways in eukaryotes are widely conserved, and removal of poly-A by deadenylase is considered to be an initial step and a rate-limiting step. Deadenylases are known to exist in a plurality of types, are broadly divided from a structure of enzyme active domain and categorized into two families of Asp-Glu-Asp-Asp (DEDD) and Exonuclease-endonuclease-phosphatase (EEP). A cap structure of the mRNA from which the poly-A strand has been removed is subsequently removed by Decapping 1/Decapping 2 (DCP1/DCP2) complex which is a decapping enzyme (Nature, 1996, Vol. 382, p. 642-646, Nucleic Acids Research, 2014, Vol. 42, p. 5217-5233). After that, the mRNA is degraded from the 5' terminal by Exoribonuclease (XRN), or subjected to degradation by exosome from the 3' terminal (Gene, 1990, Vol. 95, p. 85-90, Mol. Cell, 2004, Vol. 15, p. 173-183, Cell, 1997, Vol. 91, p. 457-466, Cell, 2007, Vol. 131, p. 1340-1353).

So far, using an antisense oligonucleotide designed against the PAS sequence, gene expression of a virus, a decrease in production amount of virus by an oligonucleotide that prevents polyadenylation at intron 9 of a KCNH2 gene (Patent Literature 1) or an antisense oligonucleotide designed for HIVPAS (Non-Patent Literature 1), or the like has been reported.

In suppression of target RNA expression by antisense nucleotides, administration of high doses of antisense nucleotides is required since it is necessary to bind the antisense nucleotides to the target RNA at a ratio of 1:1 to cause steric hindrance. Therefore, there is required a technology capable of suppressing RNA expression with high efficiency.

Eukaryotes have a mechanism referred to as RNA editing by single-base mutation. In many cases, the mechanism is site-specifically and precisely edited. For RNA editing, an adenosine deaminase enzyme (adenosine deaminase acting on RNA (ADAR) that converts adenosine (A) to inosine (I) is known. In addition, it has been reported that an ADAR2dd, a variant of an ADAR2, which is a subtype of ADAR, has cytidine deaminase activity that converts cytidine (C) to uridine (U) (Science, 2019, Vol. 365, p. 382-386).

The ADAR is a multidomain protein and has a double-stranded-RNA binding domain on an N-terminal and a deaminase domain at a C-terminal. A recognition domain recognizes a specific double strand RNA (dsRNA) sequence and/or structure, and the deaminase domain converts adenosine (A) at a specific position in a target to inosine (I) by deamination reaction of a nucleic acid base. Inosine converted from adenosine is recognized as guanine by a cellular translation mechanism. That is, in a case where the adenosine converted to inosine is in an encoding region of an mRNA or precursor mRNA, it is possible to introduce mutations into the protein sequence. Until now, it is known that there exist three types of ADARs, and expression distributions in a biological body are different. The ADAR1 and the ADAR2 are expressed throughout the body, and in particular, the ADAR2 is expressed in nerve cells. On the other hand, it has been reported that an ADAR3 is expressed only in nerve cells, has an arginine-rich single-stranded-RNA binding domain on the N-terminal side, but does not have RNA editing activity, and conversely suppresses RNA editing activity (RNA, 2000, Vol. 6, p. 755-767).

In recent years, as a target RNA editing technology using wild-type ADARs and artificial guide RNAs, several guide RNAs for editing target RNAs including ADAR-recruiting base sequences derived from GluR2 have been reported (International Publication No. 2016/097212, International Publication No. 2017/050306, International Publication No. 2017/010556, International Publication No. 2019/111957, Nucleic Acids Research, 2017, Vol. 45, p. 2797-2808, Nature Methods, 2019, Vol. 16, p. 239-242).

In addition, as a target RNA editing technology using antisense oligonucleotides, a technology of editing a target RNA by forming a double-strand of mRNA and an incomplete helix structure and recruiting an ADAR, using antisense oligonucleotides of 35 bases or more complementary to mRNA including adenosine, which is an editing target, been reported (International Publication No. 2017/220751, International Publication No. 2018/041973, International Publication No. 2018/134301, Nat. Biotechnol., 2019, Vol. 37, p. 1059-1069, Protein Eng. Des. Sel., 2018, Vol. 1, p. 471-478, Chem. Commun., 2018, Vol. 54, p. 2377-2380). In addition, ADAR-dependent editing of a target RNA using artificial RNAs of 100 bases or more has also been reported (Nat. Biotechnol., 2019, Vol. 37, p. 1059-1069).

In addition, several technologies of recruiting ADARs to a target RNA and editing the target RNA by linking an ADAR fusion protein and a guide RNA using a protein-RNA linking system have been reported. For example, a target RNA editing technology using a linking system between a fusion protein of an ADAR1 deaminase domain-MS2 coat protein (MCP) and antisense-MS2 RNA (Protein Eng. Des. Sel., 2018, Vol. 1, p. 471-478, International Publication No. 2018/161032), a target RNA editing technology using a linking system between λ phage-derived BoxB sequence and λN protein (Nucleic Acids Research, 2016, Vol. 44, p. e157, International Publication No. 2017/050306, International Publication No. 2019/071274), and the like have been reported.

In addition, several technologies of editing a target RNA using a fusion protein of an RNA-directed Cas protein and an ADAR and a guide RNA that recruits a Cas protein have also been reported. For example, a target RNA editing technology using a dCas13-ADAR fusion protein (International Publication No. 2019/005884, International Publication No. 2019/071048) and the like have been reported.

So far, there have been examples in which antisense nucleotides against the PAS sequence were used to induce suppression of mRNA expression, but there have been no reports of suppression of expression by editing the PAS sequence on RNA.

### CITATION LIST

### PATENT LITERATURE

[Patent Literature 1] International Publication No. 2016/118118

### NON-PATENT LITERATURE

Non-Patent Literature 1: Virus Res., 2012, Vol. 169, p. 63-71

### [Summary of Invention]

### TECHNICAL PROBLEM

In order to suppress expression of a target RNA using an antisense nucleotide designed against a polyadenylation signal sequence, the antisense nucleotide and the polyadenylation signal sequence of the target RNA are required to bind at a ratio of 1:1, and thus there was a problem with the efficiency of suppression of expression of target RNA. Therefore, a technology of controlling the expression of the target RNA with high efficiency has been required. An object of the present invention is to provide a guide RNA for editing a polyadenylation signal sequence of a target RNA and a nucleic acid that encodes the guide RNA, which are expected to be useful for controlling expression of the target RNA with high efficiency.

### SOLUTION TO PROBLEM

The present inventors have intensively examined technologies for controlling expression of a target RNA with high efficiency, thereby completing the present invention by discovering a guide RNA for ADAR-dependent editing of a target RNA, the guide RNA including an antisense region complementary to a portion of the target RNA including a polyadenylation signal sequence.

That is, the present invention relates to the following [1] to [14].
[1] A guide RNA for ADAR-dependent editing of a target RNA, the guide RNA including an antisense region complementary to a portion of the target RNA including a polyadenylation signal sequence.
[2] The guide RNA according to [1], in which the polyadenylation signal sequence is a base sequence consisting of AAUAAA or a base sequence consisting of a modified sequence of the base sequence.
[3] The guide RNA according to [2], in which the polyadenylation signal sequence is a base sequence consisting of AAUAAA.
[4] The guide RNA according to any one of [1] to [3], in which the antisense region complementary to a portion of the target RNA includes a base that forms a base pair with an adenosine included in the polyadenylation signal sequence of the target RNA.
[5] The guide RNA according to any one of [1] to [4], in which the ADAR is a naturally occurring ADAR, a modified ADAR, or a fusion protein of an ADAR or modified ADAR and another factor.
[6] A system of editing a target RNA, including: the guide RNA according to any one of [1] to [5] and an ADAR.
[7] A nucleic acid that encodes the guide RNA according to any one of [1] to [5].
[8] An expression vector including: a nucleic acid that encodes the guide RNA according to any one of [1] to [5].
[9] The expression vector according to [8], further including: a nucleic acid that encodes an ADAR.
[10] A host cell into which the expression vector according to [8] or [9] is introduced.
[11] A method of producing an expression vector, including: a step of culturing the host cell according to [10].
[12] A pharmaceutical composition including: the expression vector according to [8] or [9]; and a pharmaceutically acceptable excipient.
[13] A pharmaceutical composition including: the expression vector according to [8] or [9], an expression vector including a nucleic acid that encodes an ADAR, and a pharmaceutically acceptable excipient.
[14] A method of editing a target RNA, including: a step of introducing a nucleic acid that encodes the guide RNA according to any one of [1] to [5] into a cell or a virus that infects the cell.

### ADVANTAGEOUS EFFECTS OF INVENTION

A guide RNA for ADAR-dependent editing a target RNA, including an antisense region complementary to a portion of the target RNA including a polyadenylation signal sequence, can be used for controlling expression of the target RNA by editing the polyadenylation signal sequence of the target RNA. The guide RNA for ADAR-dependent editing of the target RNA can suppress a function of the polyadenylation signal sequence by binding to and editing the polyadenylation signal sequence of the target RNA and can be reused in the cell after editing, and thus the guide RNA is expected to be useful for controlling expression of the target RNA with high efficiency.

### BRIEF DESCRIPTION OF DRAWINGS

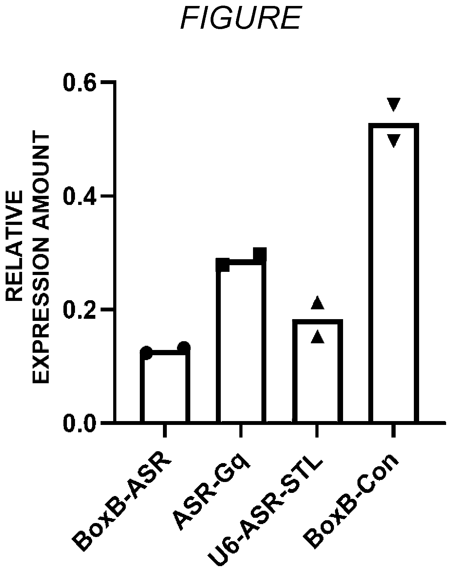

[FIGURE] The FIGURE shows a relative expression amount of Renilla Luciferase of each guide RNA with compared to a condition under which ADAR2 is not expressed. The axis of ordinate represents the relative expression amount of Renilla Luciferase compared to control. The axis of abscissa represents each of the expressed guide RNAs (BoxB-ASR, ASR-Gq, U6-ASR-STL, BoxB-Con).

### DESCRIPTION OF EMBODIMENTS

The present invention will be described in detail below, but the present invention is not limited thereto. In a case where a term used in the present specification is not specifically defined, the term is used in a sense generally accepted by those skilled in the art.

### <Guide RNA of the present invention>

The present invention provides a guide RNA for ADAR-dependent editing of a target RNA, the guide RNA including an antisense region complementary to a portion of the target RNA including a polyadenylation signal sequence (hereinafter, also referred to as "guide RNA of the present invention"). In the present invention, "a guide RNA for ADAR-dependent editing of a target RNA" refers to an RNA molecule that has a region forming a double strand with a portion of the target RNA complementary to a portion of the target RNA and recruits an ADAR to the target RNA.

### 1. Target RNA

In the present invention, "target RNA" refers to RNA that is a target of editing by the guide RNA of the present invention. In the present invention, the portion of the target RNA includes a polyadenylation signal sequence. In an aspect, the target RNA is a precursor mRNA (pre-mRNA), in an aspect, the target RNA is mRNA, in an aspect, the target RNA is a viral genomic RNA, in an aspect, the target RNA is a viral antigenomic RNA, and in an aspect, the target RNA is a virus-derived pregenomic RNA (pgRNA). The target RNA can be present in RNA included in eukaryotic cells, mammalian cells, viruses, prokaryotic cells, bacteria, phages, and the like.

### 2. Polyadenylation signal sequence

In the present invention, the target RNA includes a polyadenylation signal sequence. In the present invention, the "polyadenylation signal sequence" is a sequence known to those skilled in the art, and is a conserved motif of 6 bases present upstream 10 to 30 bases of the polyadenylation (poly (A)) site of the target RNA. In the present invention, the polyadenylation signal sequence includes adenosine that is converted to inosine. In an aspect, the polyadenylation signal sequence is a base sequence consisting of AAUAAA or a base sequence consisting of a modified sequence thereof. The modified sequence of AAUAAA is a base sequence consisting of AAUAAA, in which 1 or 2 bases are deleted, substituted, inserted and/or added, and is a base sequence recognized by cleavage-polyadenylation specificity factor (CPSF). The modified sequence of AAUAAA includes a naturally occurring modified sequence of AAUAAA or an artificially modified sequence of AAUAAA. In an aspect, the polyadenylation signal sequence is a base sequence consisting of AAUAAA and a base sequence consisting of a modified sequence thereof. In an aspect, the modified sequence of AAUAAA is AAUAAA, AGUAAA, or UAUAAA (Gene & Development, 2011, Vol. 25, p. 1770-1782). In an aspect, the polyadenylation signal sequence is a base sequence consisting of AAUAAA, AUUAAA, AGUAAA, or UAUAAA, in an aspect, the polyadenylation signal sequence is a base sequence consisting of AAUAAA or AUUAAA, and in an aspect, the polyadenylation signal sequence is a base sequence consisting of AAUAAA.

### 3. Antisense region

The guide RNA of the present invention includes an antisense region. In the present invention, the term "antisense region" refers to a region having a base sequence complementary to a portion of a target RNA including a polyadenylation signal sequence and consisting of sequences that form a double strand with the target RNA. In an aspect, the antisense region refers to a region including a base that forms a base pair with an adenosine included in the polyadenylation signal sequence of the target RNA and consisting of sequences that form a double strand with the target RNA. A length of the base sequence constituting the antisense region is, in an aspect, 10 to 100 bases, 10 to 80 bases, 10 to 60 bases, 10 to 40 bases, in an aspect, 15 to 40 bases, in an aspect, 10 to 38 bases, in an aspect, 15 to 38 bases, and in an aspect, 18 to 38 bases. In an aspect, the antisense region may include bases that form a mismatched base pair with a portion of the target RNA or bases that form a fluctuating base pair. In an aspect, the antisense region may include bases that form a mismatched base pair with a polyadenylation signal sequence of the target RNA or bases that form a fluctuating base pair. In an aspect, the antisense region may include bases that form a mismatched base pair with the polyadenylation signal sequence of the target RNA.

The "mismatched base pair" in the present specification refers to G-A, C-A, U-C, A-A, G-G, C-C, and U-U base pairs. The "fluctuating base pair" in the present specification refers to G-U, I-U, I-A, and I-C base pairs. In an aspect, the antisense region is a region having a base that forms a mismatched base pair with adenosine included in a portion of the target RNA and consisting of base sequences that form a double strand with the target RNA. In an aspect, the antisense region is a region having a base that forms a mismatched base pair with adenosine included in a polyadenylation signal sequence of the target RNA and consisting of a base sequence that forms a double strand with the target RNA. In an aspect, the antisense region is a region having cytidine which is a base that forms a mismatched base pair with adenosine included in the polyadenylation signal sequence of the target RNA, and consisting of a base sequence that forms a double strand with the target RNA.

The base sequence that constitutes the antisense region can be appropriately designed by those skilled in the art, taking into consideration the sequence of the target RNA, the base length, the position of mismatched bases formed with the target RNA, off-target effects, and the like. The base sequence that constitutes the antisense region can be designed to form a mismatched base pair or fluctuating base pair with the adenosine or cytidine, in order to improve an editing efficiency of adenosine of the polyadenylation signal sequence of the target RNA by adenosine deaminase enzyme (adenosine deaminase acting on RNA (ADAR)) (RNA, 2001, Vol. 7, p. 846-858, Nat. Biotechnol., 2019, Vol. 37, p. 1059-1069).

The "ASR" in the present specification means an antisense region.

### 4. Guide RNA for ADAR-dependent editing of target RNA

Various forms of guide RNAs are known to those skilled in the art as guide RNAs for ADAR-dependent editing of a target RNA. As long as the guide RNA of the present invention includes an antisense region complementary to a portion of the target RNA including the polyadenylation signal sequence, any guide RNA known to those skilled in the art for ADAR-dependent editing of the target RNA can be used. In an aspect, the guide RNA of the present invention includes any guide RNA of the following 1) to 5) as a guide RNA for ADAR-dependent editing of a target RNA sequence;
1) a guide RNA consisting of an antisense region (International Publication No. 2017/220751, International Publication No. 2018/041973, International Publication No. 2018/134301, Nat. Biotechnol., 2019, Vol. 37, p. 1059-1069, Protein Eng. Des. Sel., 2018, Vol. 1, p. 471-478, Chem. Commun., 2018, Vol. 54, p. 2377-2380),
2) a guide RNA consisting of an antisense region, a functional region, and any included linker sequence (International Publication No. WO2021/117729),
3) a guide RNA including an antisense region and a region that recruits ADARs (International Publication No. 2016/097212, International Publication No. 2017/050306, International Publication No. 2017/010556, International Publication No. 2019/111957, Nucleic Acids Research, 2017, Vol. 45, p. 2797-2808, Nature Methods, 2019, Vol. 16, p. 239-242, International Publication No. WO2021/020550),
4) a guide RNA including an antisense region and a region that recruits a fusion protein of ADAR and dCas13 protein (International Publication No. 2019/005884, International Publication No. 2019/071048), or
5) a guide RNA including an antisense region and a region linked to a fusion protein of ADAR and an RNA-linking protein (Protein Eng. Des. Sel., 2018, Vol. 1, p. 471-478, International Publication No. 2018/161032, Nucleic Acids Research, 2016, Vol. 44, p. e157, International Publication No. 2017/050306, International Publication No. 2019/071274).

In an aspect, the guide RNA of the present invention is a guide RNA consisting of an antisense region, a functional region, and any included linker sequence of the above 2).

In a case where the guide RNA of the present invention is the 1) guide RNA consisting of an antisense region, in an aspect, the guide RNA of the present invention is a guide RNA consisting of a region described in 3. Antisense region of <Guide RNA of the present inventions

In a case where the guide RNA of the present invention is the guide RNA consisting of an antisense region, a functional region, and any included linker sequence of the above 2), in an aspect, the guide RNA of the present invention is a guide RNA, in which a functional region is linked to the antisense region and is substantially free of ADAR-recruiting base sequences. In the present invention, "substantially free of" ADAR-recruiting base sequences (described later) means that ADAR-recruiting base sequences are not included in the same molecule of the guide RNA.

The "functional region" refers to a region consisting of base sequences having any one or more of functions of stabilization of a guide RNA, localization of the guide RNA to the nucleus, localization of the guide RNA to the cytoplasm, promotion of double strand formation between the target RNA and the antisense region, inhibition of non-specific double strand formation by the antisense region, stabilization of a complex formed by a target RNA and an antisense region. In an aspect, the guide RNA of the present invention includes, as a functional region, (a) a region consisting of a base sequence promoting stabilization of the guide RNA, (b) a region consisting of a base sequence promoting localization of the guide RNA to the nucleus, (c) a region consisting of a base sequence promoting localization of the guide RNA to cytoplasm, (d) a region consisting of a base sequence promoting a double strand formation between the target RNA and the antisense region, (e) a region consisting of a base sequence inhibiting non-specific double strand formation by the antisense region, or (f) a region consisting of a base sequence promoting stabilization of a complex formed by the target RNA and the antisense region, or a region consisting of a base sequence having any two or more functions of the functions described in the (a) to (f). A functional region having one or more of these functions may be collectively referred to as "having a function". In addition, having the functions described in (a), (b), and (d) may be collectively referred to as "having a function of an snRNA sequence".

In the present specification, "promoting stabilization of the guide RNA" means imparting resistance to RNA-degrading enzyme. The function can be evaluated by measuring a residual amount of the guide RNA in the presence of RNA-degrading enzyme, a residual amount of the guide RNA in cells under transcription inhibition after introduction of nucleic acid that encodes the guide RNA into cells, and the like, by a known method. "Promoting localization of guide RNA to nucleus" refers to promoting translocation and localization of a guide RNA introduced into a cell to a nucleus. The function can be evaluated by detecting the intracellular distribution or amount of the guide RNA by a known method. For example, it can be confirmed by in situ hybridization (Mol. Ther., 2003, Vol. 7, p. 237-247). "Promoting localization of the guide RNA to the cytoplasm" means that the guide RNA introduced into cells promotes retention in the cytoplasm. The function can be evaluated by detecting the intracellular distribution or amount of the guide RNA by a known method. For example, it can be confirmed by in situ hybridization (Mol. Ther., 2003, Vol. 7, p. 237-247). "Promoting a double strand formation of the target RNA and the antisense region" means improving the affinity between the target RNA and the antisense region. The function can be evaluated by affinity assay by a known method (BMC Biotech., 2008, Vol. 8, article number 48). "Inhibiting non-specific double strand formation by an antisense region" refers to reducing non-specific double strand formation (also referred to as "off-target effects" in the present specification). The function can be evaluated by a known method such as RNA sequencing and the like (Nat. Biotechnol., 2019, Vol. 37, p. 657-666). "Promoting stabilization of a complex formed by the target RNA and the antisense region" means that a state of the complex including a double strand formed by a target RNA and an antisense region is maintained. The function can be evaluated by measuring a residual amount of double strands formed by the target RNA and the antisense region in the presence of an RNA-degrading enzyme that degrades RNA of a DNA/RNA hybrid strand by a known method. For example, evaluation can be performed by measuring the residual amount of double strands formed by the target RNA and a guide RNA consisting of DNA sequences in the presence of RNase H (Antiviral Chemistry & Chemotherapy, 1996, Vol. 7, p. 86-93).

In an aspect, the region consisting of base sequences that promote stabilization of the guide RNA is a region consisting of a base sequence that forms a thermodynamically stabilized higher-order structure. The higher-order structure to be thermodynamically stabilized is not particularly limited as long as it is a structure that shows resistance to nuclease activity, in an aspect, the region consisting of base sequences promoting stabilization of the guide RNA, a region consisting of small nuclear RNA (snRNA) sequences, a region consisting of ribosomal RNA (rRNA) sequences, a region consisting of base sequences forming a duplex structure, a region consisting of base sequences forming a triplex strand structure, a region consisting of base sequences forming a quadruplex strand structure, a region consisting of base sequences forming a stem-loop structure, and the like. In an aspect, the region consisting of base sequences promoting stabilization of the guide RNA is a region consisting of snRNA sequences, a region consisting of base sequences forming a G-quadruplex (Gq:G-quadruplex) structure, or a region consisting of base sequences forming a stem-loop structure. In an aspect, the region consisting of base sequences promoting localization of the guide RNA to nucleus is a region consisting of small RNA-derived base sequences. In an aspect, the region consisting of base sequences promoting localization of the guide RNA to nucleus is a region consisting of snRNA sequences. In an aspect, the region consisting of base sequences promoting localization of the guide RNA to the cytoplasm is a region consisting of small RNA-derived base sequences. In an aspect, the region consisting of base sequences promoting localization of the guide RNA to the cytoplasm is a region consisting of base sequences promoting localization of the guide RNA to the ribosome, and, in an aspect, is a region consisting of rRNA sequences. In an aspect, the region consisting of base sequences promoting localization of the guide RNA to the cytoplasm is a region consisting of transfer RNA (tRNA) sequences. In an aspect, the region consisting of base sequences promoting localization of the guide RNA to the cytoplasm is a region consisting of the 7SL RNA sequences which is a Signal Recognition Particle (SRP)-derived RNA sequences. In an aspect, the region consisting of base sequences promoting double strand formation between the target RNA and the antisense region is a region consisting of base sequences thermodynamically and conformationally promoting formation of a double strand and a complex including a double strand (Nat. Biotechnol., 2019, Vol. 37, p. 657-666). In an aspect, the region consisting of base sequences promoting a double strand formation between the target RNA and the antisense region is a region consisting of snRNA sequences, a region consisting of base sequences forming a stem-loop structure, or a combination of any of these. In an aspect, the region consisting of base sequences promoting a double strand formation between the target RNA and the antisense region is a region consisting of U6 snRNA sequences, a region consisting of U1 snRNA sequences, a region consisting of U7 snRNA sequences, a region consisting of base sequences forming a stem-loop structure, or a combination of any of these. In an aspect, the region consisting of base sequences inhibiting non-specific double strand formation by the antisense region is a region consisting of base sequences thermodynamically and conformationally reducing off-target effects. In an aspect, the region consisting of base sequences inhibiting a non-specific double strand formation by the antisense region is a region consisting of base sequences forming a stem-loop structure (Chem. Commun., 2018, Vol. 54, p. 2377-2380). In an aspect, the region consisting of base sequences promoting stabilization of the complex formed between the target RNA and the antisense region is a region consisting of base sequences showing resistance to nuclease activity by thermodynamically and conformationally stabilizing a double strand and a complex including a double strand (Antiviral Chemistry & Chemotherapy, 1996, Vol. 7, pp. 86-93). In an aspect, the region consisting of base sequences promoting stabilization of the complex formed between the target RNA and the antisense region is a region consisting of base sequences forming a stem-loop structure.

In an aspect, the guide RNA of the present invention includes, as a functional region, a region consisting of snRNA sequences, a region consisting of rRNA sequences, a region consisting of base sequences forming a G-quadruplex (Gq:G-quadruplex) structure, a region consisting of base sequences forming a stem-loop structure, a region consisting of SRP-derived RNA sequences, a region consisting of long non-coding RNA (lncRNA) sequences, a region consisting of tRNA sequences, a region consisting of small nucleolar RNA (snoRNA) sequences, a region consisting of miRNA sequences, or a combination of any of these. In an aspect, the guide RNA of the present invention includes, as a functional region, a region consisting of snRNA sequences, a region consisting of rRNA sequences, a region consisting of base sequences forming a Gq structure, a region consisting of base sequences forming a stem-loop structure, or a combination of any of these.

In an aspect, the guide RNA of the present invention includes, as a functional region, a region consisting of snRNA sequences. In an aspect, the guide RNA of the present invention includes, as a functional region, a region consisting of snRNA sequences and a region consisting of base sequences forming a stem-loop structure. In an aspect, the region consisting of base sequences forming a stem-loop structure that can be used in combination with a region consisting of snRNA sequences is a region consisting of artificial stem-loop sequences. The artificial stem-loop sequence is a sequence that forms an artificially prepared stem-loop structure, and examples thereof include an artificial stem-loop sequence (referred to as "STL sequence" in the present specification) included in an expression cassette (hereinafter, the expression cassette described herein is also referred to as "U6 cassette") including a sequence encoding U6 described in Gene Ther., 1997, Vol. 4, p. 45-54, Nat. Biotechnol., 2002, Vol. 20, p. 505-508, or Mol. Ther., 2003, Vol. 7, p. 237-247. In an aspect, the region consisting of base sequences forming a stem-loop structure that can be used in combination with a region consisting of snRNA sequences is a region consisting of STL sequences. In an aspect, the guide RNA of the present invention includes, as a functional region, a region consisting of snRNA sequences and a region consisting of artificial stem-loop sequences. In an aspect, the guide RNA of the present invention includes, as a functional region, a region consisting of snRNA sequences and a region consisting of STL sequences. The region consisting of snRNA sequences used in the present invention is a region consisting of base sequences of an snRNA well known to those skilled in the art, as long as the region has a function of the snRNA sequence, the region may include a partially modified base sequence of natural snRNA, and/or as long as the region has a function of the snRNA sequence, the region may be a partial sequence of the base sequence of natural snRNA. For example, the guide RNA of the present invention includes, as a functional region, a region consisting of U1 snRNA sequences, U2 snRNA sequences, U4 snRNA sequences, U5 snRNA sequences, U6 snRNA sequences, or U7 snRNA sequences, or a combination of any of these. In an aspect, the guide RNA of the present invention includes, as a functional region, a region consisting of U6 snRNA sequences. The region consisting of U6 snRNA sequences used in the present invention is a region consisting of base sequences of the U6 snRNA, as long as the region has a function of an snRNA sequence, the region may include a partially modified base sequence of the natural U6 snRNA, and/or as long as the region has a function of the snRNA sequence, the region may be a partial sequence of the natural U6 snRNA. In an aspect, the region consisting of the U6 snRNA sequences used in the present invention is a region consisting of partial sequences of the base sequence of the U6 snRNA, and in an aspect, is a region consisting of base sequences from a transcription initiation point of the base sequence of the U6 snRNA to the 27th base. In the guide RNA of the present invention, the region consisting of the U6 snRNA sequences may be used in combination with the region consisting of the artificial stem-loop sequences as long as the region has a function of the snRNA sequence. In a case of using the region consisting of U6 snRNA sequences and the region consisting of artificial stem-loop sequences, an antisense region is inserted between the region consisting of the U6 snRNA sequences and the region consisting of the artificial stem-loop sequences. In an aspect, the region consisting of the U6 snRNA sequences used in the present invention consists of a base sequence shown in SEQ ID NO: 3, or a base sequence in which 1 to 3 bases are deleted, substituted, inserted, and/or added in a base sequence shown in SEQ ID NO: 3, having a function of the snRNA sequence. In an aspect, the region consisting of artificial stem-loop sequences used in the present invention is an STL sequence, and in an aspect, is a base sequence shown in SEQ ID NO: 5, or consists of a base sequence in which 1 to 3 bases are deleted, substituted, inserted, and/or added in a base sequence shown in SEQ ID NO: 5, forming a stem-loop structure.

In an aspect, the guide RNA of the present invention includes, as a functional region, a region consisting of base sequences forming a G-quadruplex structure, a region consisting of base sequences forming a stem-loop structure, or a combination thereof. In an aspect, the guide RNA of the present invention includes, as a functional region, a region consisting of base sequences forming a G-quadruplex structure. In an aspect, the guide RNA of the present invention includes, as a functional region, a region consisting of base sequences forming a stem-loop structure.

The region consisting of base sequences forming a G-quadruplex structure (Gq structure) used in the present invention includes a Gq sequence. The Gq sequence is a sequence including four repeating units consisting of consecutive guanines, and the four guanines form a planar quadruplex (Gq) structure. A base other than guanine may be included between repeating units consisting of guanines as long as the Gq structure is maintained. In an aspect, the Gq sequence includes repeat units consisting of 1, 2, 3, or 4 consecutive guanines, and the repeating units form 1, 2, 3, or 4 layers of Gq, respectively. In an aspect, the Gq sequence includes repeating units consisting of three consecutive guanines, and the repeating units form three layers of Gq. This is called "three-layered G-quadruplex structure" (hereinafter, also referred to as "3Gq"). In an aspect, the guide RNA of the present invention includes, as a functional region, a region consisting of base sequences forming 3Gq, and in an aspect, the base sequence forming 3Gq consists of a base sequence shown in SEQ ID NO: 9 or a base sequence in which 1 to 3 bases are deleted, substituted, inserted and/or added in the base sequence shown in SEQ ID NO: 9, and consists of base sequences forming a Gq structure.

The stem-loop structure is also referred to as a hairpin structure, and is well known in the technical field. Examples of the base sequence forming a stem-loop structure include an aptamer sequence (Int. J. Biochem. Mol. Biol., 2013, Vol. 4, p. 27-40, International Publication No. 2016/143700), a BoxB sequence-derived sequence, an MS2 sequence-derived sequence, a PP7 sequence-derived sequence (Integr. Biol., 2009, Vol. 1, p. 499-505, Nucleic Acids Research, 2016, Vol. 44, p. 9555-9564), artificial stem-loop sequence (for example, artificial stem-loop sequence (STL sequence) included in the U6 cassette (Gene Ther., 1997, Vol. 4, p. 45-54, Nat. Biotechnol., 2002, Vol. 20, p. 505- 508, Mol. Ther., 2003, Vol. 7, p. 237-247)), and the like. In an aspect, the guide RNA of the present invention includes, as a functional region, a region consisting of base sequences forming a stem-loop structure, and the region consisting of base sequences forming the stem-loop structure is a region consisting of a BoxB sequence-derived sequence, an aptamer sequence, an MS2 sequence-derived sequence, a PP7 sequence-derived sequence, or an artificial stem-loop sequence (for example, STL sequence), or a combination of any of these. In an aspect, the guide RNA of the present invention includes, as a functional region, a region consisting of the BoxB sequence-derived sequences. In an aspect, the guide RNA of the present invention includes, as a functional region, a region consisting of STL sequences. In an aspect, the region consisting of the BoxB sequence-derived sequences used in the present invention consists of a base sequence shown in SEQ ID NO: 11 or a base sequence in which 1 to 3 bases are deleted, substituted, inserted, and/or added in the base sequence shown in SEQ ID NO: 11, forming a stem-loop structure.

Among the guide RNAs of the present invention, in the guide RNA consisting of an antisense region, a functional region, and any included linker sequence of the above 2), the functional region is linked to the antisense region. The "linking" in the present specification includes a direct binding and a binding via a linker. In an aspect, in the guide RNA of the present invention, at least one functional region is directly linked to the antisense region. In an aspect, the guide RNA of the present invention has at least one functional region linked to the antisense region via a linker. In the guide RNA of the present invention, in a case where a plurality of functional regions are linked and used, the linking between the functional regions may be either a direct binding or a linking via a linker.

In the linker is used, in an aspect, the length of the linker is 1 to 10 bases, in an aspect, is 1 to 6 bases, in an aspect, is 1 to 3 bases, in an aspect, is 3 to 6 bases, in an aspect, is 3 bases, and in an aspect, is 6 bases. The base sequence of the linker can be appropriately designed by those skilled in the art based on the sequence that constitutes the guide RNA. For example, the base sequence of the linker used in the present invention may be designed so as not to form a complementary strand with the target RNA. In an aspect, the linker consists of a base sequence "UCU", "GUCGAC" which is a restriction enzyme SalI site sequence, or "UCUAGA" which is a restriction enzyme XbaI site sequence.

In an aspect, in a case where the guide RNA of the present invention includes, as a functional region, a region consisting of BoxB sequence-derived sequences, linking can be used between the region consisting of BoxB sequence-derived sequences and the antisense region via a linker, and in an aspect, the linker is a base sequence "UCU". In an aspect, in a case where the guide RNA of the present invention includes a region consisting of U6 snRNA sequences and a region consisting of base sequences forming any contained stem-loop structure as a functional region, the antisense region can be linked to the functional region via a linker, and in an aspect, the linker is a base sequence "GUCGAC" or "UCUAGA".

In an aspect, the guide RNA of the present invention is a guide RNA that includes at least one functional region and an antisense region complementary to a portion of the target RNA including a polyadenylation signal sequence and forming a double strand with the target RNA, and is substantially free of an ADAR-recruiting base sequence in which at least one of the functional regions is linked to the antisense region, having the following features:
(a-1) the guide RNA consists of a functional region consisting of U6 snRNA sequences, an antisense region, any included linker, and a region consisting of artificial stem-loop sequences, and the region consisting of U6 snRNA sequences, the antisense region, and the functional region consisting of base sequences forming an artificial stem-loop sequence structure are linked from a 5' side to a 3' side in this order,
(a-2) the guide RNA consists of an antisense region, a functional region consisting of base sequences forming a G-quadruplex structure, and any included linker, and the functional region consisting of the antisense region and the base sequence forming a G-quadruplex structure are linked from a 5' side to a 3' side in this order,
(a-3) the guide RNA consists of a functional region consisting of base sequences forming a stem-loop structure, an antisense region, and any included linker, and the functional region consisting of base sequences forming a stem-loop structure and the antisense region are linked from a 5' side to a 3' side in this order.

In the guide RNA including the antisense region and the ADAR-recruiting region of the above 3), the "ADAR-recruiting region" is a region consisting of base sequences capable of recruiting naturally occurring or modified ADARs. In an aspect, the base sequence capable of recruiting ADAR is a base sequence capable of forming a stem-loop structure in the same molecule, binding ADAR to the stem-loop structure, and recruiting the ADAR to the target RNA. Examples of the base sequence capable of recruiting ADARs include base sequences designed based on an ADAR substrate-derived stem-loop structure such as mRNA precursor of GluR2 (International Publication No. 2016/097212, International Publication No. 2017/050306, German Patent No. 102015012522, International Publication No. 2017/010556, International Publication No. 2019/111957, Nucleic Acids Research, 2017, Vol. 45, p. 2797-2808, Nature Methods, 2019, Vol. 16, p. 239-242) and the like. The guide RNA including an ADAR-recruiting region is known to those skilled in the art and can be prepared based on the description in the literature.

In the guide RNA including the antisense region and the region recruiting a fusion protein of ADAR and a dCas13 protein of the above 4), dCas13 is a modified Cas13 protein, and a modified Cas13 protein that does not have double-stranded-DNA cleavage activity. The "region recruiting a fusion protein of ADAR and dCas13 protein" is a region consisting of base sequences capable of recruiting a fusion protein of a naturally occurring ADAR or modified ADAR and a dCas13 protein. In an aspect, the region recruiting a fusion protein of an ADAR and a dCas13 protein is a base sequence capable of recruiting dCas13. The base sequence capable of recruiting dCas13 is known to those skilled in the art, and for example, is the sequence described in International Publication No. 2019/005884 or International Publication No. 2019/071048. In an aspect, the region that recruits a fusion protein of an ADAR and a dCas13 protein is a region consisting of base sequences capable of recruiting a dCas13 protein, the sequence is known to those skilled in the art, and the guide RNA including the region can be, for example, prepared based on the description in the above literature.

In the guide RNA including the antisense region and the region linked to a fusion protein of an ADAR and an RNA-linking protein of the above 5), the "RNA-linking protein" is a protein having a high affinity for a specific RNA and capable of maintaining a state of being linked to the specific RNA in a cell. In an aspect, the RNA-linking protein is an MS2 coat protein (MCP) or a λN protein. The "region linked to a fusion protein of an ADAR and an RNA-linking protein" is a region consisting of base sequences capable of linking a fusion protein of a naturally occurring ADAR or modified ADAR and an RNA-linking protein. In an aspect, the region linked to a fusion protein of an ADAR and an RNA-linking protein is a region consisting of RNA sequences having a high affinity to a specific RNA-linking protein and capable of maintaining a state of being linked to the fusion protein in a cell. The region linked to a fusion protein of an ADAR and an RNA-linking protein is an MS2 RNA in a case where the RNA-linking protein is MCP, and is a BoxB sequence in a case where the RNA-linking protein is a λN protein. The MCP, the MS2 RNA, the λN protein, or the BoxB sequence are all sequences known to those skilled in the art and are the sequences described in Protein Eng. Des. Sel., 2018, vol. 1, p. 471-478, International Publication No. 2018/161032, Nucleic Acids Research, 2016, Vol. 44, p. e157, International Publication No. 2017/050306, and International Publication No. 2019/07127.

The guide RNA of the present invention can be synthesized using a known standard polynucleotide synthesis method known in the field, based on sequence information. In addition, once the guide RNA of the present invention is obtained, it is possible to prepare a variant of the guide RNA of the present invention, in which a recruiting function to a target RNA and a function of editing the target RNA are maintained, by introducing mutations at a predetermined site using a method known to those skilled in the art such as a site-specific mutation induction method (Current Protocols in Molecular Biology edition, 1987, John Wiley & Sons Section) and the like. The guide RNA of the present invention can also be produced using modified nucleic acids. The guide RNA of the present invention can also be prepared using a nucleic acid that encodes the guide RNA of the present invention. For example, the guide RNA of the present invention can be prepared by transcribing the guide RNA of the present invention from an expression vector including a nucleic acid that encodes the guide RNA of the present invention.

### <ADAR used in the present invention>

ADARs used in the present invention include a naturally occurring ADAR, a modified ADAR, and a fusion protein of an ADAR or modified ADAR and another factor.

In an aspect, the ADARs used in the present invention also include a naturally occurring ADAR, and a modified ADAR as long as long as the ADAR has deaminase activity. Deaminase activity can be measured by detecting deamination of the substrate by methods known to those skilled in the art. For example, deaminase activity can be measured by detecting the conversion of adenosine to inosine. In an aspect, the ADAR used in the present invention is a eukaryote-derived ADAR, in an aspect, is a mammalian-derived ADAR, and in an aspect, is a human ADAR. In an aspect, the ADAR used in the present invention is an ADAR1 or ADAR2, and in an aspect, is an ADAR2. The ADAR1 includes two types of splicing variants ADAR1 p110 and ADAR1 p150, and in an aspect, the ADAR used in the present invention is an ADAR1 p110 or ADAR1 p150. In an aspect, the ADAR used in the present invention is a human ADAR1 or human ADAR2, and in an aspect, is a human ADAR2. In an aspect, the ADAR used in the present invention is a polypeptide including a double-stranded-RNA binding domain (dsRBD) and having deaminase enzymatic activity (Trends in Biochemical Sciences, 2001, Vol. 26, p. 376-384, RNA, 2001, Vol. 7, p. 846-858). In an aspect, the ADAR used in the present invention is a polypeptide including a deaminase domain and capable of converting adenosine of the target RNA to inosine. The ADAR used in the present invention may be a fusion protein of an ADAR or modified ADAR and another factor.

In an aspect, the ADAR used in the present invention is a polypeptide consisting of an amino acid sequence shown in Accession No. [NP_001103.1], Accession No. [NP_056648.1], or Accession No. [NP_001102.3]; or a polypeptide consisting of an amino acid sequence having 90% or more identity to these amino acid sequences, or an amino acid sequence in which 1 to 10 amino acids are deleted, substituted, inserted and/or added in these amino acid sequences and having adenosine deaminase activity. In an aspect, the ADAR used in the present invention is a polypeptide consisting of an amino acid sequence (human ADAR2) shown in SEQ ID NO: 15, or a polypeptide consisting of an amino acid sequence having 90% or more identity to the sequence, or an amino acid sequence in which 1 to 10 amino acids in the sequence are deleted, substituted, inserted and/or added and having adenosine deaminase activity. In an aspect, the ADAR used in the present invention may be an ADAR that is endogenously present in a eukaryotic cell, and in an aspect, may be an ADAR that is exogenously introduced into the eukaryotic cell. The introduction of the ADAR into a eukaryotic cell may be direct introduction of ADAR polypeptides or may be introduction of expression vectors including a nucleic acid that encodes an ADAR.

In the present specification, the term "identity" means a value identity obtained by using parameters prepared by default by EMBOSS NEEDLE program (J. Mol. Biol., 1970, Vol. 48, p. 443-453) search. The above parameters are as follows.
Gap Open Penalty = 10
Gap Extend Penalty = 0.5
Matrix = EBLOSUM62

In an aspect, the ADAR used in the present invention is a fusion protein of a naturally occurring or modified ADAR and another factor. Here, in an aspect, another factor includes a dCas13 protein or an RNA-linking protein. In an aspect, the ADAR used in the present invention is a fusion protein of a naturally occurring ADAR or modified ADAR and dCas13. The fusion protein is a protein consisting of an amino acid sequence known to those skilled in the art, for example, a protein consisting of the amino acid sequence described in International Publication No. 2019/005884 or International Publication No. 2019/071048. In an aspect, the ADAR used in the present invention is a fusion protein of a naturally occurring or modified ADAR and an RNA-linking protein. In an aspect, the ADAR used in the present invention is a fusion protein of a naturally occurring ADAR or modified ADAR and MCP or a fusion protein of a naturally occurring ADAR or modified ADAR and a λN protein. The fusion protein is a protein consisting of an amino acid sequence known to those skilled in the art, for example, a protein consisting of amino acid sequences described in Protein Eng. Des. Sel., 2018, vol. 1, p. 471-478, International Publication No. 2018/161032, Nucleic Acids Research, 2016, Vol. 44, p. e157, International Publication No. 2017/050306, and International Publication No. 2019/071274.

### <System of editing target RNA of the present invention>

The invention also provides a system of editing a target RNA including the guide RNA and the ADAR of the present invention. A system of editing a target RNA sequence including the guide RNA and the ADAR of the present invention includes a kit for editing a target RNA sequence including the guide RNA and the ADAR of the present invention, or a method of editing a target RNA sequence using the guide RNA and the ADAR of the present invention. In the present invention, "editing" refers to converting a base of a target RNA sequence into a different base. The system of editing a target RNA of the present invention can be used intracellularly or extracellularly. In an aspect, the system can be used in eukaryotic cells. In an aspect, the system can be used in prokaryotic cells, bacteria, phages, viruses, and the like.

### <Nucleic acid that encodes the guide RNA of the present invention>

The present invention also provides a nucleic acid that encodes the guide RNA of the present invention (also referred to as "nucleic acid of the present invention" in this section). The nucleic acid of the present invention is a nucleic acid that encodes a guide RNA for ADAR-dependent editing of a target RNA, the guide RNA including an antisense region complementary to a portion of the target RNA including a polyadenylation signal sequence.

In an aspect, the nucleic acid of the present invention is a nucleic acid that encodes a guide RNA for ADAR-dependent editing of a target RNA, the guide RNA including an antisense region complementary to a portion of the target RNA including a polyadenylation signal sequence, in which the polyadenylation signal sequence is a base sequence consisting of AAUAAA or a modified sequence thereof. In an aspect, the nucleic acid of the present invention is a nucleic acid that encodes a guide RNA for ADAR-dependent editing of a target RNA, the guide RNA including an antisense region complementary to a portion of the target RNA including a polyadenylation signal sequence, in which the polyadenylation signal sequence is a base sequence consisting of AAUAAA. In an aspect, the nucleic acid of the present invention is a nucleic acid that encodes a guide RNA for ADAR-dependent editing of a target RNA, the guide RNA including an antisense region complementary to a portion of the target RNA including a polyadenylation signal sequence, in which the antisense region complementary to a portion of the target RNA includes a base that forms a base pair with an adenosine included in the polyadenylation signal sequence of the target RNA. In an aspect, the nucleic acid of the present invention is a nucleic acid that encodes a guide RNA for ADAR-dependent editing of a target RNA, the guide RNA including an antisense region complementary to a portion of the target RNA including a polyadenylation signal sequence, and recruiting a naturally occurring ADAR, a modified ADAR, or a fusion protein of an ADAR or modified ADAR and another factor.

The nucleic acid of the present invention can be synthesized using a standard polynucleotide synthesis method known in the field, based on the sequences described in the present specification or publicly available sequence information. In addition, once a nucleic acid of the present invention is obtained, it is possible to prepare other nucleic acids of the present invention by introducing mutations into a predetermined site using a method known to those skilled in the art such as a site-specific mutation induction method (Current Protocols in Molecular Biology edition, 1987, John Wiley & Sons Section) and the like.

### <Expression vector including nucleic acid that encodes the guide RNA of the present invention>

The present invention also provides an expression vector (also referred to as an "expression vector of the present invention") including a nucleic acid that encodes a guide RNA of the present invention. The present invention also provides an expression vector including a nucleic acid that encodes a guide RNA of the present invention and a nucleic acid that encodes an ADAR. The nucleic acid that encodes the guide RNA of the present invention and the nucleic acid that encodes the ADAR may be mounted on the same expression vector, or may be mounted on a separate expression vector. In an aspect, the expression vector of the present invention is a combination of an expression vector including a nucleic acid that encodes the guide RNA of the present invention and an expression vector including a nucleic acid that encodes an ADAR. In an aspect, the expression vector of the present invention is an expression vector including a nucleic acid that encodes the guide RNA of the present invention and a nucleic acid that encodes an ADAR.

### 1. Expression vectors used in the present invention

The expression vector used in the present invention is not particularly limited as long as the expression vector is an expression vector capable of expressing the guide RNA of the present invention from a nucleic acid that encodes the guide RNA of the present invention and/or an expression vector capable of expressing an ADAR. In an aspect, the expression vector used in the present invention is an expression vector that can be used for expressing the guide RNA of the present invention and/or for expressing an ADAR in human cells. In an aspect, examples of the expression vector used in the present invention include a plasmid vector, a viral vector (for example, an adenoviral vector, a retroviral vector, a lentiviral vector, an adeno-associated viral vector), and the like.

### 2. Promoter

The expression vector of the present invention may include a promoter operably linked to a nucleic acid that encodes the guide RNA of the present invention and/or a nucleic acid that encodes an ADAR. In the present specification, "operably linked" means that at least one promoter is linked to a nucleic acid such that a polypeptide or RNA encoded by the nucleic acid can be expressed in a host cell. The promoter included in the expression vector of the present invention is not particularly limited, but a promoter corresponding to an RNA polymerase (for example, RNA polymerase II (pol II) and RNA polymerase III (pol III)) suitable for expressing a nucleic acid that encodes the guide RNA or the ADAR of the present invention can be used. As an aspect, in order to express the guide RNA of the present invention, it is possible to use a promoter corresponding to the pol II or pol III, and as another aspect, it is possible to use a promoter compatible with the pol III. As an aspect, in order to express an ADAR, it is possible to use a promoter compatible with the pol II.

Examples of the pol II-compatible promoter include a cytomegalovirus (CMV)-derived promoter, a simian virus 40 (SV40) promoter, a respiratory syncytial virus (RSV) promoter, an Elongation factor 1α (EF1 α) promoter, a CAG (CMV enhancer, chicken beta-Actin promoter) promoter, a U1 snRNA promoter, a U7 snRNA promoter, and the like. Examples of the pol III-compatible promoter include a human U6 snRNA promoter (U6) (Nat. Biotechnol., 2002, vol. 20, p. 497-500), a highly sensitive U6 promoter (Nucleic Acids Research, 2003, vol. 31, p. e100), a human H1 promoter, a 5S rRNA promoter, and other viral and eukaryotic cell promoters known to those skilled in the art, but are not limited thereto.

The expression vector of the present invention may further include a translation initiation codon, a translation termination codon, a purine base (G or A) preferable for the transcription initiation point of pol III, a poly-A signal, a continuous T terminator sequences for the pol III, an enhancer, an untranslated region, a splicing junction, and the like, depending on the promoter, host cell, or the like to be used.

### <Host cell of the present invention>

The present invention also provides a host cell transformed by introducing a nucleic acid that encodes the guide RNA of the present invention (also referred to as a "host cell of the present invention"). In an aspect, the host cell of the present invention is a host cell into which the expression vector of the present invention is introduced. In an aspect, the host cell of the present invention is a host cell into which the expression vector of the present invention, which is a plasmid vector, is introduced. In an aspect, the host cell of the present invention is a host cell into which a plasmid vector for producing the expression vector of the present invention, which is a viral vector, is introduced. In an aspect, the host cell of the present invention is a host cell into which the expression vector of the present invention, which is a viral vector, is introduced.

The host cell into which the expression vector of the present invention is introduced is not particularly limited, but any cell known in the field can be selected as long as the cell can be used to produce the expression vector of the present invention. Examples of the host cell that can be used for vector replication include various cells such as natural cells, artificially established cells commonly used in the technical field of the present invention, or the like. Examples of the host cell that can be used for vector replication include animal cells (for example, CHO cells, HEK293 cells, and the like), insect cells (for example, Sf9 and the like), bacteria (for example, E. coli and the like), yeasts (genus Saccharomyces, Pichia, and the like), and the like. In an aspect, E. coli can be used as a host cell. Transformation can be performed by methods known to those skilled in the art (Green, M.R. and Sambrook, J., Molecular Cloning: A Laboratory Manual, 4th Edition, Cold Spring Harbor Laboratory Press, 2012).

### <Method of producing the expression vector of the present invention>

The method of producing the expression vector of the present invention includes a method of producing a nucleic acid including a step of culturing a host cell into which an expression vector including the nucleic acid of the present invention is introduced. In an aspect, the method of producing the expression vector of the present invention includes a step of culturing a host cell into which the expression vector of the present invention is introduced to replicate the expression vector of the present invention. In a case where the expression vector of the present invention is a viral vector, the method of producing an expression vector of the present invention includes a step of culturing a host cell into which a viral vector plasmid including a nucleic acid of the present invention is introduced, and purifying the viral vector prepared in the host cell. The viral vector can be prepared by methods known to those skilled in the art.

The method of producing the expression vector of the present invention may include a step of collecting a culture solution of the host cell to obtain a lysate. The lysate can be, for example, obtained by treating the collected culture solution with an alkali dissolution method or a boiling method. The method of producing the expression vector of the present invention may further include a step of purifying the expression vector from the lysate. In purification of the expression vector from the lysate, an ion exchange chromatography and/or hydrophobic interaction chromatography can be used. In a case where the expression vector of the present invention is a viral vector, in purification of the viral vector from the lysate, a cesium chloride density gradient centrifugation method, a sucrose gradient centrifugation method, an iodixanol density gradient centrifugation method, a limit extrafiltration method, a diafiltration method, an affinity chromatography method, an ion exchange chromatography method, a polyethylene glycol precipitation method, an ammonium sulfate precipitation method, or the like can be used.

### <Pharmaceutical composition of the present invention>

The present invention also provides a pharmaceutical composition (also referred to as "pharmaceutical composition of the present invention") including the guide RNA of the present invention, a nucleic acid that encodes the guide RNA of the present invention (also referred to as "nucleic acid of the present invention"), or the expression vector of the present invention, and a pharmaceutically acceptable excipient. In an aspect, the pharmaceutical composition of the present invention is a pharmaceutical composition including the guide RNA of the present invention and a pharmaceutically acceptable excipient. In an aspect, the pharmaceutical composition of the present invention is a pharmaceutical composition including the guide RNA of the present invention, an ADAR, and a pharmaceutically acceptable excipient. In an aspect, the pharmaceutical composition of the present invention includes a pharmaceutical composition including the nucleic acid of the present invention and a pharmaceutically acceptable excipient. In an aspect, the pharmaceutical composition of the present invention is a pharmaceutical composition including the nucleic acid of the present invention, an ADAR, and a pharmaceutically acceptable excipient. In an aspect, the pharmaceutical composition of the present invention is a pharmaceutical composition including an expression vector including a nucleic acid that encodes the guide RNA of the present invention and a pharmaceutically acceptable excipient. In an aspect, the pharmaceutical composition of the present invention is a pharmaceutical composition including an expression vector including a nucleic acid of the present invention and a nucleic acid that encodes an ADAR, and a pharmaceutically acceptable excipient. In an aspect, the pharmaceutical composition of the present invention is a pharmaceutical composition including an expression vector including a nucleic acid that encodes the guide RNA of the present invention, an expression vector including a nucleic acid that encodes an ADAR, and a pharmaceutically acceptable excipient.

The pharmaceutical composition of the present invention can be prepared by a generally used method using an excipient generally used in the field, that is, a pharmaceutical excipient, a pharmaceutical carrier, and the like. Examples of dosage forms of these pharmaceutical compositions include a parenteral agent such as an injection agent and a drip agent and the like, and can be administered by intravenous administration, subcutaneous administration, intradermal administration, intramuscular administration, and the like. In formulating, excipients, carriers, additives, and the like can be used according to these dosage forms within a pharmaceutically acceptable range

The guide RNA of the present invention, the nucleic acid of the present invention, the expression vector of the present invention, the expression vector including a nucleic acid that encodes an ADAR, or the dosage of an ADAR is determined by the degree of symptoms and age of the patient, the dosage form of the preparation to be used, and the like, for example, the dosage in a range of 0.001 mg/kg to 100 mg/kg.

In an aspect, a disease that can be prevented or treated with the pharmaceutical composition of the present invention is any disease in which transcriptional control of the target RNA by editing one or more adenosines in the polyadenylation signal sequence of the target RNA results in beneficial changes. The pharmaceutical composition of the present invention can be used as a prophylactic or therapeutic agent for any disease in which transcriptional control of the target RNA by editing of one or more adenosines in the polyadenylation signal sequence of the target RNA results in beneficial changes.

The present invention includes a prophylactic or therapeutic pharmaceutical composition for any disease in which transcriptional control of the target RNA by editing of one or more adenosines in the polyadenylation signal sequence of the target RNA including the guide RNA of the present invention, the nucleic acid of the present invention, or the expression vector of the present invention results in beneficial changes. In addition, the present invention also includes a method of preventing or treating any disease in which transcriptional control of the target RNA by editing of one or more adenosines in the polyadenylation signal sequence of the target RNA results in beneficial changes, including a step of administering a prophylactically or therapeutically effective amount of the guide RNA of the present invention, the nucleic acid of the present invention, or the expression vector of the present invention. In addition, the present invention also includes the guide RNA of the present invention, the nucleic acid of the present invention, or the expression vector of the present invention, for use in the prevention or treatment of any disease in which transcriptional control of the target RNA by editing of one or more adenosines in the polyadenylation signal sequence of the target RNA results in beneficial changes. In addition, the present invention includes use of the guide RNA of the present invention, the nucleic acid of the present invention, or the expression vector of the present invention in production of a prophylactic or therapeutic pharmaceutical composition for any disease in which transcriptional control of the target RNA by editing of one or more adenosines in the polyadenylation signal sequence of the target RNA results in beneficial changes.

### <Method of editing the target RNA of the present invention>

The present invention also provides a method of editing a target RNA using the guide RNA of the present invention (also referred to as "editing method of the present invention"). In an aspect, the editing method of the present invention includes a method in which the antisense region of the guide RNA of the present invention forms a double strand with a portion of the target RNA including the polyadenylation signal sequence, the ADAR is recruited to the guide RNA of the present invention, and the recruited ADAR converts adenosine of the polyadenylation signal sequence of the target RNA sequence to inosine. Here, the adenosine on the polyadenylation signal sequence of the target RNA converted to inosine may form a mismatched base pair with the antisense region.

The editing method of the present invention includes step (i) of introducing the guide RNA of the present invention, a nucleic acid that encodes the guide RNA of the present invention, or an expression vector of the present invention into a cell having the target RNA, a virus that infects the cell having a target RNA, or the like. In an aspect, the editing method of the present invention further includes step (ii) of introducing an ADAR, a nucleic acid that encodes an ADAR, or an expression vector including a nucleic acid that encodes an ADAR into the cell, a virus that infects the cell, or the like. Steps (i) and (ii) may be performed simultaneously or separately. In an aspect, the cells to be introduced are eukaryotic cells, prokaryotes, and the like, and in an aspect, the cells are mammalian cells, bacteria, or the like. In an aspect, the virus that infects the introduced cells includes a phage. In an aspect, adenosines in the polyadenylation signal sequence of the target RNA are converted to inosines.

### Examples

Unless otherwise specified, the steps described in the following examples can be implemented according to known methods. In addition, for parts using commercially available kits, reagents, or the like, experiments were performed according to the attached protocol unless otherwise specified.

### <Example 1 Preparation of guide RNA expression plasmid including antisense region and functional region (U6 snRNA sequence and STL sequence)>

A vector obtained by substituting an H1 RNA polymerase III promoter (hereinafter, referred to as "H1 promoter") of the pSUPER. neo vector (Oligoengine, Inc., Catalog No. VEC-PBS-0004) to the human U6 RNA polymerase III (hU6) promoter sequence (SEQ ID NO: 1) was constructed (hereinafter, referred to as "pSUPER. neo-U6 vector"). A fragment including the hU6 promoter sequence was amplified using a standard PCR method using Tks Gflex^{™} DNA Polymerase (Takara Bio Inc., Catalog No. R060A) so as to add each restriction enzyme site of EcoRI on a 5' side and BglII on a 3' side, having pBAsi-hU6 Neo DNA (Takara Bio Inc., Catalog No. 3227) having a hU6 promoter as a template. The obtained hU6 promoter fragment was inserted into the pSUPER. neo vector using restriction enzymes EcoRI site (5' side) and BglII site (3' side). E. coli DH5α Competent Cells (Takara Bio Inc., Catalog No. 9057, hereinafter, referred to as "DH5α E. coli strain") were transformed with the constructed pSUPER. neo-U6 vector and subjected to liquid culture. The culture solution was centrifuged to collect bacterial cells, the plasmid extraction and purification were performed with NucleoBond (registered trademark) Xtra Midi Plus EF (Takara Bio Inc., Catalog No. U0422B), and the pSUPER. neo-U6 vector was amplified.

An expression plasmid of the guide RNA added with the antisense region and the functional region U6 snRNA sequence (hereinafter, sometimes referred to as "U6") was constructed as follows. A DNA sequence (SEQ ID NO: 2) encoding U6-ASR-STL which is a guide RNA was inserted downstream the hU6 promoter of the pSUPER. neo-U6 vector, using restriction enzymes BGIII site (5' side) and HindIII site (3' side). In preparing the DNA sequence to be inserted, a method of annealing forward oligos and reverse oligos (described below) synthesized to form a restriction enzyme-cleaved terminal was used.
Forward oligo: DNA sequence -3' encoding 5'-GATC-guide RNA
Reverse oligo: Reverse complementary sequence-3' of DNA sequence encoding 5'-AGCTT-guide RNA

The inserted DNA sequence includes a purine base (G or A) preferable for the pol III transcription initiation point on the 5' side and the pol III terminator sequence on the 3' side. The prepared guide RNA expression plasmid is referred to as pSUPERneo_U6_gRNA. Here, ASR is an antisense base sequence consisting of 33 bases complementary to a portion including AATAAA (PAS) present in an artificial PAS sequence (1688-1736 of NCBI Accession No. AY535007.1) attached to a posterior portion of Renilla Luciferase (Rluc) mRNA of psiCHECK-2 vector (Promega Corporation, Catalog No. C8021), and consists of RNA sequences (also referred to as ASR) encoded by sequences from a 35th base to a 67th base, out of the DNA sequence shown in SEQ ID NO: 2. The U6 consists of RNA sequence shown in SEQ ID NO: 3, and the nucleic acid that encodes the U6 consists of DNA sequences shown in SEQ ID NO: 4. The STL consists of the RNA sequence shown in SEQ ID NO: 5, and the nucleic acid that encodes the STL consists of the DNA sequence shown in SEQ ID NO: 6.

Table 1 shows an overview of the present guide RNA. The constructed pSUPERneo_U6_gRNA plasmid was amplified in the same manner as described above using DH5α E. coli strain (Takara Bio Inc., Catalog No. 9057).

### <Example 2 Preparation of guide RNA expression plasmid including antisense region and functional region (Gq sequence- or BoxB sequence-derived sequence)>

The expression plasmid of the guide RNA including an antisense region and a functional region (Gq sequence- or BoxB sequence-derived sequence) was prepared as follows. The DNA sequences (SEQ ID NO: 7 and SEQ ID NO: 8, respectively) encoding ASR-Gq or BoxB-ASR, which are guide RNAs, were inserted downstream the H1 promoter of the pSUPER. neo vector using restriction enzymes BglII (5' terminal side) and HindIII (3' terminal side). The DNA sequence encoding the inserted guide RNA includes a purine base (G or A) preferable for the transcription initiation point of the pol III on the 5' terminal side and the pol III terminator sequence on the 3' terminal side. The Gq sequence (hereinafter, also referred to as Gq) consists of the RNA sequence shown in SEQ ID NO: 9, and the nucleic acid that encodes Gq consists of the DNA sequence shown in SEQ ID NO: 10. The BoxB sequence-derived sequence (hereinafter, also referred to as BoxB) consists of the RNA sequence shown in SEQ ID NO: 11, and the nucleic acid that encodes BoxB consists of the DNA sequence shown in SEQ ID NO: 12. The DNA sequence to be inserted was prepared using the same method as in Example 1. The obtained guide RNA expression plasmids are collectively referred to as pSUPERneo_H1_gRNA. The constructed plasmid was amplified by the same method as in Example 1 using the DH5α E. coli strain. The expression plasmid of the guide RNA (referred to as BoxB-Con) used for comparison control was prepared by the same method as described above, using a DNA sequence (SEQ ID NO: 13) added with the DNA sequence encoding BoxB to the DNA sequence encoding the ASR sequence (RNA sequences encoded to sequences from a 30th base to a 64th base shown in SEQ ID NO: 13. Also referred to as Con-ASR) having SV40PAS sequence as a target. The inserted DNA sequence includes a purine base (G or A) preferable for the pol III transcription initiation point on the 5' side and the pol III terminator sequence on the 3' side.

Table 1 shows an overview of each guide RNA prepared in Examples 1 and 2 and the DNA sequence encoding thereof. In Table 1, "5' terminal" indicates a functional base sequence added to the 5' terminal side of ASR, and "3' terminal" indicates a functional sequence added to the 3' terminal side of ASR. The "linker" indicates a sequence linking ASR and each functional base sequence.

**[Table 1]**

| Guide RNA name | SEQ ID NO (DNA) | Functional sequence | 5' terminal | Linker | ASR | Linker | 3' terminal |
|---|---|---|---|---|---|---|---|
| U6-ASR-STL | SEQ ID NO: 2 | U6 | U6 | GUCGAC | ASR | UCUAGA | STL |
| ASR-Gq | SEQ ID NO: 7 | Gq | - | - | ASR | - | Gq |
| BoxB-ASR | SEQ ID NO: 8 | BoxB | BoxB | UCU | ASR | - | - |
| BoxB-Con | SEQ ID NO: 13 (control) | BoxB | BoxB | UCU | Con-ASR | - | - |

### <Example 3 Preparation of expression plasmid for detecting editing activity of intracellular target RNA>

A psiCHECK-2_Rluc-bGHpoly A vector carrying a Rluc gene, which is used as a plasmid for detecting the editing activity of target RNA in cells, was prepared by the following method. A bovine Growth Hormone (hereinafter, bGH) of an artificial PAS sequence was incorporated into the 3' terminal side of the Rluc gene PAS sequence carried in the psiCHECK-2 vector. An artificially synthesized DNA sequence (SEQ ID NO: 14) with a restriction enzyme NotI site added to the 5' terminal side and a SacII site added to the 3' terminal side was inserted using NotI and SacII. The constructed vector was referred to as the psiCHECK-2_Rluc-bGHpoly A vector, and this vector was amplified by the same method as in Example 1 using the DH5α E. coli strain.

### <Example 4 Preparation of ADAR2 expression plasmid, which is an RNA editing enzyme>

A pAAV-CMV-ADAR2 plasmid, which is an ADAR2 expression plasmid, was prepared by inserting a DNA sequence (SEQ ID NO: 16) encoding the ADAR2 (SEQ ID NO: 15) downstream a CMV-derived promoter of a pAAV-CMV vector (Takara Bio Inc., Catalog No. 6230) using restriction enzymes EcoRI (5'terminal side) and BglII (3' terminal side). SEQ ID NO: 16 includes a Kozak sequence ("ACC" from a 13th base to a 15th base of SEQ ID NO: 16) upstream the translation initiation codon, and a termination codon downstream the ADAR2 gene. The constructed pAAV-CMV-ADAR2 plasmid was amplified by the same method as in Example 1 using the DH5α E. coli strain.

### <Example 5 Transfection and Sanger sequencing>

Human embryonic kidney-derived cell line HEK293 cells were suspended in 5% fetal bovine serum (FBS)-added Dulbecco's Modified Eagle Medium (DMEM, high glucose, GlutaMAXtm Supplement, pyruvate; ThermoFisher Scientific, Catalog No. 10569-010), seeding was performed at 2.0 × 10⁴ cells/100 µL on a 96-well plate, and cultured overnight under a condition of 37°C in the presence of 5% CO₂.

The following (1) to (4) were mixed at a weight ratio of 1 : 40 : 30 : 9 so that a total amount was 100 ng/well, and transfected into HEK293 cells cultured overnight after seeding using Lipofectamine^{™} 3000 Transfection Reagent (ThermoFisher Scientific, Catalog No. L3000015).
(1) Expression plasmid for detecting intracellular target RNA editing activity prepared in Example 3 (psiCHECK-2_Rluc-bGHpolyA vector)
(2) ADAR2 expression plasmid (pAAV-CMV-ADAR2) prepared in Example 4 or pBluescript II KS (-) Phagemid Kit (Agilent Technologies, Catalog No. 212208) as a plasmid for correcting an amount of transfection (also referred to as pBSKS)
(3) Guide RNA expression plasmid (pSUPERneo_U6_gRNA and pSUPERneo_H1_gRNA) prepared in Examples 1 and 2, and
(4) pHelper Vector (Takara Bio Inc., Catalog No. 6230) as a carrier plasmid.

After transfection, the cells were cultured under a condition of 37°C for 3 days in the presence of 5% CO₂, and then the cells were collected and subjected to examination of RNA editing efficiency by Sanger sequencing as described in Example 6.

The transfected cells were collected, and total RNA was extracted and purified according to an attached protocol using QIAshredder (QIAGEN, Catalog No. 79656) from the cells, RNeasy Mini Kit (QIAGEN, Catalog No. 74106), and RNase-free DNase Set (QIAGEN, Catalog No. 79254). The obtained RNA was subjected to reverse transcription reaction using SuperScript (registered trademark) VILO^{™} cDNA Synthesis kit (ThermoFisher Scientific, Catalog No. 11754-250) according to the attached protocol to obtain cDNA. PCR reaction was performed having cDNA as a template and using the following primers and Prime STAR (registered trademark) GXL DNA Polymerase (Takara Bio Inc., Catalog No. R050A) for amplifying a fragment including an editing point according to the attached protocol. The primers used in the experiments in Table 2 are described as follows.
Forward primer: AGCGGGAATGGCTCATATCG (SEQ ID NO: 17)
Reverse primer: GGCACCTTCCAGGGTCAAG (SEQ ID NO: 18)
Sequence primer: GGAGAAGGGCGAGGTTAGAC (SEQ ID NO: 19)

The PCR amplified fragment was purified using ExoSAP-IT^{™} Express PCR Cleanup Reagents (Thermofisher Scientific, Catalog No. 75001.200.UL) according to the attached protocol, and subjected to Sanger sequencing reaction by a 3730xl DNA Analyzer (Applied Biosystems) together with sequencing primers.

Table 2 shows the editing efficiency at an A position of a 4th base in the sequence of AATAAA, which is a representative editing position, among the results obtained in transfection experiments.

The column of ADAR2 in the table represents the presence or absence of transfection of the ADAR2 expression plasmid, ADAR2+ represents cells added with pAAV-CMV-ADAR2 (hereinafter, also referred to as "ADAR2+ cells"), and ADAR2- represents cells added with pBSKS (hereinafter, also referred to as "ADAR2-cells"). The column of RNA editing rate (%) represents a ratio (G/(G + A) × 100) of signal intensity of guanine (G), which analyzed a waveform data file (extension: .ab1) obtained by Sanger sequencing reaction with QSVanalyzer software (Bioinformatics, 2009, Vol. 25, p3244-3250).

**[Table 2]**

| Guide RNA name | ADAR2 | RNA editing rate (%) |
|---|---|---|
| U6-ASR-STL | ADAR2- | 1.3 |
| ASR-Gq | | 1.2 |
| BoxB-ASR | | 1.0 |
| BoxB-Con | | 2.5 |
| U6-ASR-STL | ADAR2+ | 7.7 |
| ASR-Gq | | 13.3 |
| BoxB-ASR | | 6.3 |
| BoxB-Con | | 2.3 |

As shown in Table 2, in BoxB-Con, which is a guide RNA for comparison, the RNA editing rate was almost constant (2.5 vs. 2.3) regardless of the presence or absence of ADAR transfection. BoxB-ASR, ASR-Gq, and U6-ASR-STL, which are guide RNAs with a functional sequence added to the antisense region having the PAS sequence as a target, showed an increase in the RNA editing rate under conditions of ADAR2 transfection.

### <Example 6 Examination of mRNA amount of target RNA by transfection and Quantitative PCR>

psiCHECK-2 vector, ADAR2 expression plasmid (pAAV-CMV-ADAR2) prepared in Example 4, guide RNA expression plasmids prepared in Examples 1 and 2 (pSUPERneo_U6_gRNA, pSUPERneo_H1_gRNA), and pHelper Vector of carrier plasmid were mixed so that a total amount was 100 ng/well at a weight ratio of 1 : 40 : 30 : 9, respectively. HEK293 cells cultured overnight after seeding were transfected using Lipofectamine^{™} 3000 Transfection Reagent. After transfection, the cells were cultured under a condition of 37°C in the presence of 5% CO₂ for 3 days, and the cells were collected and subjected to quantitative PCR examination. Quantitative PCR was performed from the transfected cells using the TaqMan (registered trademark) Gene Expression Cells-to-CT^{™} Kit (ThermoFisher Scientific, Catalog No. AM1728). After washing the cells with PBS, a cell solution was prepared using a 50 µl of solution in which Lysis buffer and DNase I were mixed, and cDNA was synthesized according to the protocol using 22.5 µl of the prepared cell solution. After that, Quantitative PCR was performed using human 18S (ThermoFisher Scientific, Catalog No. Hs999999901_s1) or Rluc primers and probes shown below.
Forward primer for Rluc: CTTCTTAGCTCCCTCGACAATAG (SEQ ID NO: 20)
Reverse primer for Rluc: TCCAGATTGTCCGCAACTAC (SEQ ID NO: 21)
Probe for Rluc: CCAGCGACGATCTGCCTAAGATGTT (SEQ ID NO: 22)

The analysis used a delta-delta Ct method. 18S, which is a housekeeping gene, was used as an endogenous control for correction between samples. The axis of ordinate in the FIGURE indicates a relative expression amount under ADAR2 expression, and test results of two independent trials are shown in respective plots. As shown in the FIGURE, it was recognized that compared to BoxB-Con, which is a guide RNA for comparison, the guide RNA with a functional sequence added to an ASR region having the PAS sequence as a target has a lower expression ratio of mRNA of Rluc which is a target.

### INDUSTRIAL APPLICABILITY

A pharmaceutical composition including the guide RNA of the present invention, a nucleic acid that encodes the guide RNA of the present invention, and the expression vector of the present invention is useful for suppressing expression of a target RNA.

### SEQUENCE LISTING FREE TEXT

The numerical heading <223> in the following sequence listing describes the description of "Artificial Sequence". SEQ ID NOs: 1-2, 4, 6-8, 10, 12-14, and 16-22 are synthetic DNA sequences; SEQ ID NOs: 3, 5, 9, and 11 are synthetic RNA sequences; and SEQ ID NO: 15 is a protein sequence.

## Claims

1. A guide RNA for ADAR-dependent editing of a target RNA,
wherein the guide RNA includes an antisense region complementary to a portion of the target RNA including a polyadenylation signal sequence.

2. The guide RNA according to Claim 1,
wherein the polyadenylation signal sequence is a base sequence consisting of AAUAAA or a base sequence consisting of a modified sequence of the base sequence.

3. The guide RNA according to Claim 2,
wherein the polyadenylation signal sequence is a base sequence consisting of AAUAAA.

4. The guide RNA according to any one of Claims 1 to 3,
wherein the antisense region complementary to a portion of the target RNA includes a base that forms a base pair with an adenosine included in the polyadenylation signal sequence of the target RNA.

5. The guide RNA according to any one of Claims 1 to 4,
wherein the ADAR is a naturally occurring ADAR, a modified ADAR, or a fusion protein of an ADAR or modified ADAR and another factor.

6. A system of editing a target RNA, comprising:
the guide RNA according to any one of Claims 1 to 5; and
an ADAR.

7. A nucleic acid that encodes the guide RNA according to any one of Claims 1 to 5.

8. An expression vector comprising:
a nucleic acid that encodes the guide RNA according to any one of Claims 1 to 5.

9. The expression vector according to Claim 8, further comprising:
a nucleic acid that encodes an ADAR.

10. A host cell into which the expression vector according to Claim 8 or 9 is introduced.

11. A method of producing an expression vector, comprising:
a step of culturing the host cell according to Claim 10.

12. A pharmaceutical composition comprising:
the expression vector according to Claim 8 or 9; and
a pharmaceutically acceptable excipient.

13. A pharmaceutical composition comprising:
the expression vector according to Claim 8 or 9;
an expression vector including a nucleic acid that encodes an ADAR; and
a pharmaceutically acceptable excipient.

14. A method of editing a target RNA, comprising:
a step of introducing a nucleic acid that encodes the guide RNA according to any one of Claims 1 to 5 into a cell or a virus that infects the cell.
